# EUROPEAN PATENT APPLICATION

(11) **EP 0 867 181 A1**
(43) Date of publication of application: **30.09.1998**
(21) Application number: 97200920.3
(22) Date of filing: 26.03.1997
(51) Int. Cl.: A61K 31/40, A61K 9/00, A61K 47/48

(54) **Nasal melatonin composition**

(71) Applicant: Merkus, Franciscus Wilhelmus Henricus Maria, B-2460 Kasterlee (BE)
(72) Inventor: Merkus, Franciscus Wilhelmus Henricus Maria, B-2460 Kasterlee (BE)
(74) Representative: Louet Feisser, Arnold

(57) **Abstract**

The invention relates to a pharmaceutical composition for nasal administration comprising melatonin and an additive selected from glycerol, cyclodextrin, and mixtures thereof. The pharmaceutical composition comprising an aqueous composition of 0.5 to 10 mg/ml of melatonin, and one or more of 1 to 250 mg/ml of cyclodextrin and 5 to 50% by volume of glycerol, can be used for the preparation of a nasal dosage form comprising daily dosage units of 0.05 to 1 mg of melatonin, and preferably of about 0.1 to 0.8 mg of melatonin, for sleep induction in men.

## Description

The invention relates to a pharmaceutical composition for nasal administration of melatonin. The invention further relates to dosage units for nasal administration, and the use of the same for the preparation of a nasal dosage form.

Melatonin (N-acetyl-5-methoxytryptamine) is a neurohormone secreted from the pineal gland. It has been disclosed in numerous references that melatonin induces sleep when administered to a patient. The sleep-inducing effects of melatonin have advantages over conventional hypnotics, since not being a hypnotic drug itself, it only induces a state of sleepiness without having the adverse side-effects of conventional hypnotics. Melatonin is usually administered orally, but other routes of administration, in particular nasal administration may be preferred. However, because of inherent problems, *inter alia* due to the low solubility of melatonin in water, suitable pharmaceutical compositions for nasal administration are not yet known. It has, however, been tried to develop suitable pharmaceutical compositions for nasal administration. In an early attempt Vollrath et al., Adv. Bioscience, 29 (1981), pp. 327-329 described the nasal administration of 1.7 mg of melatonin in ethanol. Due to serious local irritation and painful administration, this composition appeared to be unsuitable for application in men. In 1980 intranasal compositions with less ethanol have been disclosed in Japanese patent application J5 5057-563 (Hoechst AG). A composition of 100 mg of melatonin in 10 ml of a 5% solution of ethanol in water has been described, which gives a pharmaceutical composition which still causes adverse side-effects. This patent application also discloses a pharmaceutical composition free from ethanol. By using propylene glycol instead of ethanol, a nasal pharmaceutical composition was obtained which does not show the acute problems of ethanol, but which appears to be unsuitable because of toxicity of the propylene glycol, which adversely affects the nasal mucosa. It has, therefore, been assumed that (poly)alcohols are unsuitable as excipients in nasal compositions containing melatonin. There is, however, a need for a nasal composition comprising melatonin, in order to obtain pharmaceutical compositions having a fast onset time. It has now been found that glycerol and cyclodextrin, when applied in nasal composition containing melatonin, do not exhibit the unwanted toxic and adverse effects of the (poly)alcohols, which are known in the art as additives of nasal compositions. It has further been found that cyclodextrin accelerates the absorption of melatonin in the nasal mucosa.

This invention therefore concerns nasal compositions having a dosage which is sufficient for inducing sleep in men, and which is not toxic and does not display irritant behavior, and has a short onset time.

In a broad sense the invention pertains to a pharmaceutical composition for nasal administration comprising melatonin and an additive selected from glycerol, cyclodextrin, and mixtures thereof.

The nasal composition of the invention can be administered as nasal spray, drop, solution, suspension, gel, ointment, cream, or powder. The composition may also be administered using a nasal tampon or a nasal sponge. Preferably, the composition is administered in the form of an aqueous composition or a dry powder. The aqueous composition is preferably an aqueous solution, suspension, or gel.

When taken as an aqueous composition suitable compositions are obtained with or without glycerol as an additive. Glycerol allows aqueous compositions containing relatively high amounts of melatonin, whereas this triol does not exert toxicity towards the epithelian membrane, and does not lead to irritation of the nasal mucosa. An additional advantage of the use of glycerol is the preservative properties thereof, leading to stable solutions.

When taken as a powder, the composition preferably contains cyclodextrin to obtain a melatonin-cyclodextrin complex with optimum onset times. The term cyclodextrin refers to cyclodextrins such as α-cyclodextrin, β-cyclodextrin, γ-cyclodextrin, and derivatives thereof, such as methylated or alkylated cyclodextrins. Examples are methylated β-cyclodextrin, hydroxypropyl- and hydroxyethyl-cyclodextrin, (di)glucosyl- or (di)maltosyl-cyclodextrins, carboxymethyl- or sulfoalkylether cyclodextrins, such as sulfobutylether-β-cyclodextrin. The preferred cyclodextrin is methylated β-cyclodextrin, or more preferred β-cyclodextrin.

When a complex of melatonin and cyclodextrin is used, and additionally glycerol is used as a further additive, the ratio of glycerol:cyclodextrin may vary between 1:5 and 500:1 by weight, and preferably between 1:1 and 50:1 by weight.

It has now been found that extremely low dosages of melatonin can be administered, provided that the administration is performed through the nasal route. According to this invention a single pharmaceutical dosage unit for nasal administration containing 0.05 to 1 mg, and preferably, 0.1 to 0.8 mg of melatonin, and an additive selected from glycerol, cyclodextrin, and mixtures thereof, gives effective sleep-induction in men.

The invention therefore also relates to the use of a pharmaceutical composition comprising an aqueous composition of 0.5 to 10 mg/ml of melatonin, and one or more of 1 to 250 mg/ml of cyclodextrin and 5 to 50% by volume of glycerol, or a powder of 0.5 to 50% by weight of melatonin, and 2.5 to 90% by weight of cyclodextrin, for the preparation of a nasal powder comprising daily dosage units of 0.05 to 1 mg of melatonin, and preferably of about 0.1 to 0.8 mg of melatonin, for sleep induction in men.

Surprisingly, melatonin is very effective at this dose while toxic effects are not observed.

The invention therefore also comprises a method for inducing sleep in patients, by administering nasally a pharmaceutical composition at a unit dosage of 0.05 to 1 mg of melatonin, and preferably of about 0.1 to 0.8 mg of melatonin.

The invention also comprises the use of a pharmaceutical composition for the preparation of a nasal dosage form, such as a spray, comprising daily dosage units of 0.05 to 1 mg of melatonin, and preferably of about 0.1 to 0.8 mg of melatonin, for inducing sleep in patients,

Mixed with pharmaceutically suitable auxiliaries and liquids, e.g. as described in the standard reference, Gennaro et al., Remington's Pharmaceutical Sciences, (18th ed., Mack Publishing Company, 1990, see especially Part 8: Pharmaceutical Preparations and Their Manufacture) the compounds may be processed into vials or containers, for instance, in the form of a solution or as a spray. For making dosage units any other pharmaceutically acceptable additive which does not interfere with the function of the active compounds can be used.

To further improve the absorption of melatonin from the nasal composition, nasal absorption enhancers, which are known in the art, may be added. Also viscosity enhancers may be added, for example natural gums, cellulose derivatives such as hydroxypropylmethyl cellulose or methyl cellulose, acrylic polymers (carbopol), and vinyl polymers (polyvinylpyrrolidone). Other conventional pharmaceutically acceptable excipients may also be added, such as preservatives, surfactants, colorants, co-solvents, adhesives, anti-oxidants, buffers, and agents to adjust the pH and osmolarity.
Nasal powder compositions can be made by mixing the active ingredients and the excipients, both possessing the desired particle size. Other methods to make a suitable powder formulation are the preparation of a solution of active ingredients and excipients, followed by precipitation, filtration, and pulverization, or followed by removal of the solvent by freeze-drying, followed by pulverization of the powder to the desired particle size. The final step can be sieving to obtain particles with a size of less than 100 µm in diameter, preferably between 50 and 100 µm in diameter. Powders can be administered using a nasal insufflator, a jet-spray, or any other conventional device known in the art.

The invention is further illustrated by the following examples.

### Example 1

A solution was prepared containing:

| | |
|---|---|
| melatonin | 50 mg |
| 1:1 molar complex of melatonin and β-cyclodextrin | 900 mg |
| sodium chloride | 900 mg |
| benzalkonium chloride | 10 mg |
| sodium EDTA | 100 mg |
| sterilized water | up to 100 ml |

The solution was filtered, stored at 5°C for 24 h, and filtered again. The solution can be processed into vials or containers, for instance a container of 10 ml, containing 100 dosage units of 0.2 mg of melatonin each.

### Example 2

In a reaction vessel 200 mg of melatonin and 900 mg of sodium chloride were agitated at room temperature in a mixture of 30 ml of glycerol and water up to 100 ml, until a clear solution was obtained.
The solution was filtered, stored at 5°C for 24 h, and filtered again. This solution can be processed into vials or containers, for instance a container of 10 ml, containing 100 dosage units of 0.2 mg of melatonin each.

### Example 3

A solution was prepared containing 200 mg of melatonin, 1.2 g of a complex of 200 mg of melatonin and 1 g of β-cyclodextrin, 5 g of sorbitol, 10 ml of glycerol, and 10 mg of benzalkonium chloride in an amount of water up to a total volume of 100 ml. The solution was filtered, stored at 5°C for 24 h, and filtered again. This solution can be processed into vials or containers, for instance a container of 10 ml, containing 100 dosage units of 0.4 mg of melatonin each.

## Claims

1. A pharmaceutical composition for nasal administration comprising melatonin and an additive selected from glycerol, cyclodextrin, and mixtures thereof.

2. The pharmaceutical composition of claim 1 wherein the composition is an aqueous composition or a powder.

3. The pharmaceutical composition of claim 1 or 2 wherein the composition is a powder comprising cyclodextrin as the additive.

4. The pharmaceutical composition of claim 1 or 2 wherein the composition is an aqueous composition comprising glycerol as the additive.

5. The pharmaceutical composition of any one of claims 1-3 wherein the composition comprises β-cyclodextrin as the additive.

6. A pharmaceutical dosage unit for nasal administration containing 0.05 to 1 mg of melatonin, and preferably, 0.1 to 0.8 mg of melatonin and an additive selected from glycerol, cyclodextrin, and mixtures thereof.

7. Use of a pharmaceutical composition comprising an aqueous composition of 0.5 to 10 mg/ml of melatonin, and one or more of 1 to 250 mg/ml of cyclodextrin and 5 to 50% by volume of glycerol, for the preparation of a nasal dosage form comprising daily dosage units of 0.05 to 1 mg, and preferably of about 0.1 to 0.8 mg, for sleep induction in men.

8. Use of a pharmaceutical composition comprising a powder of 0.5 to 50% by weight of melatonin, and 2.5 to 90% by weight of cyclodextrin, for the preparation of a nasal powder comprising daily dosage units of 0.05 to 1 mg of melatonin, and preferably of about 0.1 to 0.8 mg of melatonin, for sleep induction in men.
